# EUROPEAN PATENT SPECIFICATION

(11) **EP 2 405 904 B1**
(45) Date of publication and mention of the grant of the patent: **05.12.2018**
(21) Application number: 10708983.1
(22) Date of filing: 12.03.2010
(51) Int. Cl.: A61K 9/70, A61K 49/00, A61K 38/00

(54) **METHOD OF TREATING ECZEMA**
VERFAHREN ZUR BEHANDLUNG VON EKZEM
PROCÉDÉ DE TRAITEMENT DE L'ECZÉMA

(30) Priority: 13.03.2009 EP 09155180
(43) Date of publication of application: 18.01.2012
(73) Proprietor: DBV Technologies, 92120 Montrouge (FR)
(72) Inventor: BENHAMOU, Pierre-Henri, 75116 Paris (FR); DUPONT, Christophe, 92140 Clamart (FR); MONDOULET, Lucie, 92320 Chatillon (FR); DIOSZEGHY, Vincent, 94350 Villiers Sur Marne (FR)
(74) Representative: Becker, Philippe
(86) International application number: PCT/EP2010/053219
(87) International publication number: WO 2010/103116

(56) References cited:
- WO-A2-00/16752
- US-A1- 2007 048 361
- T. WERFEL ET AL: "Usefulness of specific immunotherapy in patients with atopic dermatitis and allergic sensitization to house dust mites: a multi-centre, randomized, dose-response study.", ALLERGY, vol. 61, no. 2, 1 February 2006 (2006-02-01), pages 202-205, XP55003095, ISSN: 0105-4538, DOI: 10.1111/j.1398-9995.2006.00974.x
- MONDOULET L ET AL: "Epicutaneous Immunotherapy (EPIT) for House Dust Mite (HDM) Allergy using VIASKIN<(>R) Technology: a Preclinical Study", JOURNAL OF ALLERGY AND CLINICAL IMMUNOLOGY, MOSBY, INC, US, vol. 123, no. 2, 1 February 2009 (2009-02-01), page S177, XP027569758, ISSN: 0091-6749 [retrieved on 2009-02-01]
- MONDOULET L ET AL: "Epicutaneous immunotherapy for peanut allergy: a preclinical study", ALLERGY, MUNSKGAARD, COPENHAGEN, vol. 63, no. SUPPL. 88, 11 June 2008 (2008-06-11), pages 10-11, XP008103948, ISSN: 0105-4538, DOI: DOI:10.1111/J.1398-9995.2008.01758.X [retrieved on 2008-05-27]

## Description

### FIELD OF THE INVENTION

References in the description to a treatment or to a method of treatment refer to the compositions and patches of the present invention for use in a method of treatment. The present invention relates to the treatment of eczema. More specifically, the invention relates to a new method of treating eczema through the epicutaneous route. In particular, the method of the invention comprises applying to a non eczematous area of the skin of a subject in need thereof a skin patch device comprising a composition, under conditions allowing a contact between said composition and the skin. The present invention also relates to the skin patch device.

### BACKGROUND OF THE INVENTION

Eczema is a form of dermatitis, or inflammation of the epidermis. The term eczema is broadly applied to a range of persistent skin conditions. The terms "eczema" and "dermatitis" are used interchangeably in the present application. These include dryness and recurring skin rashes which are characterized by one or more of these symptoms: redness, skin edema (swelling), itching and dryness, crusting, flaking, blistering, cracking, oozing, or bleeding. Itchy rash is particularly noticeable on head and scalp, neck, inside of elbows, behind knees, and buttocks. There are several types of eczematous disorders, for example atopic eczema, asteatotic eczema, contact eczema, allergic contact eczema, seborrheic eczema, nummular eczema, dyshidrotic eczema, dermatitis herpetiformis, stasis dermatitis and neurodermatitis.

Atopic dermatitis (atopic eczema, AE) is an inflammatory, chronically relapsing, non-contagious and pruritic skin disease. Atopic eczema is an allergic disease believed to have a hereditary component. The skin of a patient with atopic dermatitis reacts abnormally and easily to irritants, food, and environmental allergens and becomes red, flaky and very itchy. It also becomes vulnerable to surface infections caused by bacteria. The skin on the flexural surfaces of the joints (for example inner sides of elbows and knees) are the most commonly affected regions in people.

Food allergy and atopic eczema (AE) may occur in the same patient and it is now accepted that foods, such as cow's milk, peanuts or hen's eggs, can directly provoke AE. In general, inhaled allergens such as pollen are of greater importance in older children, adolescents and adults. Adolescents and adults may also react to foods, but reactions to 'classical' food allergens, such as hen's eggs and cow's milk, are not as common as in childhood.

Histologically, three phases should be distinguished:
- acute phase: acute eczema is characterised by spongiosis, a mainly intercellular oedema in-between the epidermal keratinocytes leading to micro- or macrovesicles. It is regularly accompanied by infiltration of leukocytes, mostly lymphocytes, into the epidermis and upper dermis;
- subacute phase: spongiosis is reduced and vesicles are not present anymore. The infiltration of leukocytes, mostly lymphocytes, is diminished. The epidermis thickens and parakeratosis (retention of keratinocyte nuclei in the stratum corneum) may develop;
- chronic phase: inflammation and spongiosis may be mild or completely absent. Thickening of the epidermis is more pronounced than in the subacute phase, as acanthosis (thickening of the stratum spinosum), hyperkeratosis (thickening of the stratum corneum) and parakeratosis (dysfunction of the keratinisation) occur.

In addition eosinophils are noticed at all the stages of the eczema process in various concentration in the tissues depending on the cause of eczema.

Atopic dermatitis often occurs together with other atopic diseases like hay fever, asthma and conjunctivitis. It is a familial and chronic disease and its symptoms can increase or disappear over time. Atopic dermatitis in older children and adults is often confused with psoriasis. Atopic dermatitis afflicts humans, particularly young children; it is also a well-characterized disease in domestic dogs.

Although there is no cure for atopic eczema, and its causes not well understood, it can be treated very effectively in the short term through a combination of prevention (learning what triggers the allergic reactions) and drug therapy.

Since the beginning of the twentieth century, many mucosal inflammatory disorders have become dramatically more common; atopic eczema (AE) is a classic example of such a disease. It now affects 10-20% of children and 1-3% of adults in industrialized countries, and its prevalence in the United States alone has nearly tripled in the past thirty to forty years.

Although it is such a common disease, relatively little is understood about the underlying causes of atopic eczema (Klüken et al., 2003). While AE is associated with allergic asthma and allergic rhinitis, the connection between the diseases has not been established. Twin studies have consistently shown that the disease has a higher rate of concordance in identical as compared to fraternal twins, which also indicates that genetics plays a role in its development. However, the rate of concordance between identical twins is far from 100%, and the changing frequency of the disease over time points to the environmental factors-nutrition or hygiene, for instance-that also play a role in disease susceptibility.

Since there is no cure for atopic eczema, treatment should mainly involve discovering the triggers of allergic reactions and learning to avoid them. Werfel et al. proposed specific immunotherapy in patients with atopic dermatitis and allergic sensitization to house dust mites (Allergy 2006, 61, 202-205). Allergen was injected subcutaneously .

Originally controversial, the association of food allergy with atopic dermatitis has now been clearly demonstrated. Many common food allergens can trigger an allergic reaction: such as milk, nuts, cheese, tomatoes, wheat, yeast, soy, and corn. Many of these allergens are common ingredients in grocery store products (especially corn syrup, which is a sugar substitute). Specialty health food stores often carry products that do not contain common allergens. If a child avoids these allergens early on, the frequency of reactions to these later in life is decreased significantly. Breastfeeding is the best way to avoid these problems, but if that is unavailable, then hydrolyzed formulas are preferred to cow's milk (van Odijk et al., 2003).

Since dust is a very common allergen and irritant, adults with atopic eczema should likely avoid smoking, as well as the inhalation of dust in general. The dander from the fur of dogs and cats may also trigger an inflammatory response. It is a common misconception that simply removing an animal from a room will prevent an allergic reaction from occurring. A room must be completely free of animal dander in order to prevent an allergic reaction. Anger, stress, and lack of sleep are also factors that are known to aggravate eczema. Excessive heat (especially with humidity) and coldness are known to provoke outbreaks, as well as sudden and extreme temperature swings.

An allergy "scratch" test, given by an allergist, can often pinpoint the triggers of allergic reactions. Once the causes of the allergic reactions are discovered, the allergens should be eliminated from the diet, lifestyle, and/or environment. If the eczema is severe, it may take a lot of time for the body's immune system to begin to settle down after the irritants are withdrawn.

The primary treatment involves prevention, which includes avoiding or minimizing contact with (or intake of) known allergens. Once that has been established, topical treatments can be used. Topical treatments focus on reducing both the dryness and inflammation of the skin.

To combat the severe dryness associated with eczema, a high-quality, dermatologist approved moisturizer should be used daily. Moisturizers should not have any ingredients that may further aggravate the condition. Most commercial soaps wash away all the oils produced by the skin that normally serve to prevent drying. Using a soap substitute such as aqueous cream helps keep the skin moisturized. A non-soap cleanser can be purchased usually at a local drug store. Showers should be kept short and at a lukewarm/moderate temperature.

If moisturizers on their own don't help and the eczema is severe, topical corticosteroid ointments, creams, or injections may be applied. Corticosteroids have traditionally been considered the most effective method of treating severe eczema. Disadvantages of using steroid creams include stretch marks and thinning of the skin. Higher-potency steroid creams must not be used on the face or other areas where the skin is naturally thin; usually a lower-potency steroid is prescribed for sensitive areas. If the eczema is especially severe, prednisone or a shot of cortisone or triamcinolone may be applied.

If complications include infections (often of Staphylococcus aureus), antibiotics may be employed.

The immunosuppressants tacrolimus and pimecrolimus can be used as a topical preparation in the treatment of severe atopic dermatitis instead of or in additoin to traditional steroid creams. There can be unpleasant side effects in some patients such as intense stinging or burning, which mostly get better after the first week of treatment (Jasek et al., 2007).

A more novel form of treatment involves exposure to broad or narrow-band ultraviolet light. UV radiation exposure has been found to have a localized immunomodulatory effect on affected tissues, and may be used to decrease the severity and frequency of flares (Beattie et al., 2005). In particular, Meduri et al. have suggested that the usage of UVA1 is more effective in treating acute flares, whereas narrow-band UVB is more effective in long-term management scenarios (Meduri et al., 2007). However, UV radiation has also been implicated in various types of skin cancer, and thus UV treatment is not without risk (Jans et al., 2006).

If ultraviolet light therapy is employed, initial exposure should be no longer than 5-10 minutes, depending on skin type. UV therapy should only be moderate, and special care should be taken to avoid sunburn (sunburn will only aggravate the eczema).

Consequently, there is a need for an immunotherapy method for the treatment of eczema which is safe, efficient and well tolerated by patients.

### SUMMARY OF THE INVENTION

The present invention provides a new method of treating eczema. More specifically, the invention shows, for the first time, that efficient immunotherapy of eczema can be achieved through the epicutaneous route. In particular, the method of the invention comprises applying to a non eczematous area of the skin of a subject in need thereof a skin patch device as defined in the claims, under conditions allowing a contact between said substance and the skin. The present invention shows that an application of the skin patch device according to the invention provokes a very substantial decrease of the skin reactivity of the subject, which is not limited to the area of application of the device.

An object of this invention thus resides in a method of treating eczema in a subject, said method comprising applying to a non eczematous area of the skin of the subject a skin patch device comprising a substance that causes a cutaneous inflammatory reaction, under conditions allowing a contact between said composition and the skin.

A further object of this invention relates to an occlusive skin patch device comprising (a composition comprising) a substance that causes a cutaneous inflammatory reaction, in dry form, adhering to the patch through electrostatic forces, for use in the treatment of eczema in a subject.

The invention also relates to the use of a skin patch device comprising a substance that causes a cutaneous inflammatory reaction, in dry form, adhering to the patch through electrostatic forces, in the manufacture of a composition for treating eczema.

A further object of this invention is a method of treating eczema in a subject, the method comprising applying one or several allergens, in dry form, to one or several non eczematous areas of the skin of the subject and maintaining the allergen in contact with the skin under conditions sufficient to cause a decrease of the skin reactivity of the subject.

A further object of this invention is a method of decreasing the skin reactivity of a subject having eczema, the method comprising applying one or several allergens, in dry form, to one or several non eczematous areas of the skin of the subject and maintaining the allergen in contact with the skin under conditions sufficient to cause a decrease of the skin reactivity of the subject.

The invention may be used in any mammalian subject, particularly in human subjects.

### BRIEF DESCRIPTION OF THE DRAWINGS

**Figure 1** shows an eczematous reaction after one application of PPE in sensitized mice (left); and a lack of reactivity after 3 weeks of EPIT treatment with Viaskin®(right).
**Figure 2** shows a persistance of a skin reaction to atopy patch test in sensitized non treated guinea pigs after 3 months of treatment (left); lack of reactivity in animals treated by EPIT (right).
**Figure 3** shows that after application of OVA, macrophages and eosinophils are increased in sensitized mice (red) and have not been influenced in EPIT treated mice (green).
**Figure 4** shows the evolution of the skin reactivity during the treatment of mice sensitized to peanut proteins. A: skin reactivity after the first application of peanut-Viaskin®, B: skin reactivity after the last application of peanut-Viaskin®, C: monitoring of the skin reactivity during 8 weeks of treatment (with one 48h-Viaskin® application per week).

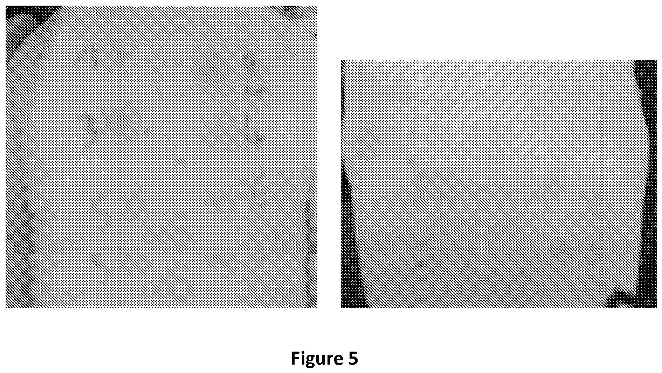

**Figure 5** shows an evolution of the skin reactivity of a human subject during the treatment (patient n°3-3, treated group) at day 30 (left) and day 90 (right). The green point indicates the last place in which the device has been removed. A: at the beginning of the treatment, a high level of reactivity has been observed on the skin with large inflammatory reactions in every location. B: after 3 months of treatment, reactions are widely attenuated.

### DETAILED DESCRIPTION OF THE INVENTION

The present invention relates to a method for treating eczema in a subject using epicutaneous administration.

In particular, the method of the invention relates to a method of treating eczema in a subject, said method comprising applying to a non eczematous area of the skin of the subject a skin patch device comprising a composition comprising a substance that causes a cutaneous inflammatory reaction under conditions allowing a contact between said composition and the skin.

In a specific aspect of the invention, the application of said skin patch device comprising said substance/allergen to the skin causes a decrease of the skin reactivity of the subject.

In another embodiment of the invention, the substance causes a cutaneous inflammatory reaction with eosinophils.

The invention advantageously shows that such a method causes a substantial decrease of the skin reactivity of the subject in a zone which is not limited to the local zone of application of the device.

The invention may be used to treat various types of eczema, including without limitation atopic eczema, asteatotic eczema, contact eczema, allergic contact eczema, seborrheic eczema, nummular eczema, dyshidrotic eczema, dermatitis herpetiformis, stasis dermatitis and neurodermatitis. In a preferred embodiment of the invention, said eczema is atopic eczema.

The substance according to the invention is an allergen, namely a milk, peanut, egg pollen or dust mite allergen.

In a particular embodiment, the method involves a repeated application of the skin patch device. In another particular embodiment, the method involves the application of at least 2 patches over a period of 1 month or more.

In another embodiment, the composition further contains a pharmaceutically acceptable carrier.

A particular object of this invention relates to an occlusive skin patch device comprising an allergen, in dry form, for treating eczema.

In a further particular embodiment, the allergen is maintained on the patch through electrostatic forces, wherein said patch is applied to the non eczematous area of the skin of the subject under conditions allowing a contact between said composition and the skin.

A further embodiment of the present invention resides in a use of an occlusive patch device described above, in the manufacture of a composition for treating eczema.

The present invention provides a new epicutaneous immunotherapy method for treating eczema, which comprises repeatedly administering to said subject a composition via the epicutaneous route by means of a skin patch device comprising a backing, the periphery of said backing being adapted to create with the skin a hermetically closed chamber, wherein the backing bears on its skin facing side within the chamber said one or more proteins in a dose sufficient to decrease the skin reactivity in said subject following application of the patch device to the skin, said composition being removed from the backing following application of the patch device to the skin and thereafter delivered to the subject via the epicutaneous route, said administration leading, on repetition, to a progressive decrease of a skin reactivity.

In another aspect, the present invention also concerns the use of a skin patch device comprising a backing, the periphery of said backing being adapted to create with the skin a hermetically closed chamber, wherein the backing bears on its skin facing side within the chamber an allergen, in the manufacture of a composition for treating eczema in a subject.

The invention may be used in any subject, for example animal or human subject, and particularly any human subject, including children and adults. Preferably, the subject has an atopic eczema.

The immunotherapeutic method of the invention involves the administration of an allergen composition to a subject via the epicutaneous route using particular patch devices, leading to decreasing the skin reactivity.

As used in this specification, the term "epicutaneous route" means the administration of an allergen to a subject by application of this allergen on the skin. The epicutaneous route does not require the use of a needle, syringe or of any other means to perforate or to alter the integrity of the superficial layer of the epidermis. The allergen is maintained in contact with the skin for period of time and under conditions sufficient to allow the allergen to penetrate into the stratum corneum of the epidermis.

The term "treating" includes a reduction of skin reactivity in patients, thereby leading to a disappearance of eczema. The allergen or a combination of allergens is selected from food allergens, contact allergens and respiratory allergens, namely from (cow's) milk, peanuts, (hen's) eggs, house dust mite and pollen.

In a preferred embodiment, the allergen composition comprises one or more proteins. The In allergen composition is in a dry form, in particular in a particulate form, obtained, for example, by lyophilisation. The use of proteins in particular dry form is advantageous. Indeed, such particulate allergens may be directly attached to the backing of the device, thereby avoiding any chemical interaction or any reaction which might disturb the immunogenicity of these proteins. Moreover, the use of the particles allows preserving the substance in a suitable packaging, such that there is no longer any need to carry out an extemporaneous preparation. In this case, the epicutaneous administration of allergens held on the backing of the patch may be ensured by dissolution of these allergens in the condensation formed within the hermetically closed chamber.

The composition used in the present invention is formulated without any adjuvant. Within the context of this invention, an adjuvant designates any substance that acts to activate, accelerate, prolong, or enhance antigen-specific immune responses when used in combination with specific antigen. Adjuvant compounds include mineral salts, such as calcium phosphate, aluminium phosphate, and aluminium hydroxide; immunostimulatory DNA or RNA, such as CpG oligonucleotides; proteins, such as antibodies or Toll-like receptor binding proteins; saponins e.g. QS21; cytokines; muramyl dipeptide derivatives; LPS; MPL and derivatives including 3D-MPL; GM-CSF (Granulocyte-macrophage colony-stimulating factor); imiquimod; colloidal particles; complete or incomplete Freund's adjuvant; Ribi's adjuvant or bacterial toxin e.g. cholera toxin or enterotoxin (LT).

The skin patch device used in the method of the invention preferably comprises a backing, the periphery of said backing being adapted to create with the skin a hermetically closed chamber. This backing bears on its skin facing side within the chamber the composition used to decrease the skin reactivity.

Preferably, the periphery of the backing has adhesive properties and forms an airtight joint to create with the skin a hermetically closed chamber.

In a particular embodiment, the composition allergens are maintained on the backing by means of electrostatic and/or Van der Waals forces. This embodiment is particularly suited where the composition allergens are in solid or dry form (e.g., particles).

Within the context of the present invention, the term "electrostatic force" generally designates any non-covalent force involving electric charges. The term Van der Waals forces designates non-covalent forces created between the surface of the backing and the solid allergen, and may be of three kinds: permanent dipoles forces, induced dipoles forces, and London-Van der Waals forces. Electrostatic forces and Van der Waals forces may act separately or together.

In this respect, in a preferred embodiment, the patch device comprises an electrostatic backing. As used herein, the expression "electrostatic backing" denotes any backing made of a material capable of accumulating electrostatic charges and/or generating Van der Waals forces, for example, by rubbing, heating or ionization, and of conserving such charges. The electrostatic backing typically includes a surface with space charges, which may be dispersed uniformly or not. The charges that appear on one side or the other of the surface of the backing may be positive or negative, depending on the material constituting said backing, and on the method used to create the charges. In all cases, the positive or negative charges distributed over the surface of the backing cause forces of attraction on conducting or non-conducting materials, thereby allowing to maintain the allergen. The particles also may be ionized, thereby causing the same type of electrostatic forces of attraction between the particles and the backing.

Examples of materials suitable to provide electrostatic backings are glass or a polymer chosen from the group comprising cellulose plastics (CA, CP), polyethylene (PE), polyethylen terephtalate (PET), polyvinyl chlorides (PVCs), polypropylenes, polystyrenes, polycarbonates, polyacrylics, in particular poly(methyl methacrylate) (PMMA) and fluoropolymers (PTFE for example). The foregoing list is in no way limiting.

The back of the backing may be covered with a label which may be peeled off just before application. This label makes it possible, for instance, to store the composition allergen in the dark when the backing is at least partially translucent.

The intensity of the force between a surface and a particle can be enhanced or lowered by the presence of a thin water film due to the presence of moisture. Generally, the patch is made and kept in a dry place. The moisture shall be low enough to allow the active ingredient to be conserved. The moisture rate can be regulated in order to get the maximum adhesion forces. As discussed above, the use of an electrostatic backing is particularly advantageous where the allergen is in a dry form, e.g., in the form of particles. Furthermore, the particle size may be adjusted by the skilled person to improve the efficiency of electrostatic and/or Van der Waals forces, to maintain particles on the support.

In a specific embodiment, the patch comprises a polymeric or metal or metal coated polymeric backing and the particles of composition allergens are maintained on the backing essentially by means of Van der Waals forces. Preferably, to maintain particles on the support by Van der Waals forces, the average size of the particles is lower than 60 micrometer. In another embodiment, the allergens are maintained on the backing by means of an adhesive coating on the backing. The backing can be completely covered with adhesive material or only in part. Different occlusive backings can be used such as polyethylene or PET films coated with aluminium, or PE, PVC, or PET foams with an adhesive layer (acrylic, silicone, etc.).

Preferred examples of patch devices for use in the present invention are disclosed in WO02/071950 or US7,635,488 (Viaskin).

Other examples are disclosed in WOO2009/095591, which also discloses a spray-drying process to load the substance in particulate form on the backing of a patch device. An electrospray device uses high voltage to disperse a liquid in the fine aerosol. Allergens dissolved in a solvent are then pulverized on the patch backing where the solvent evaporates, leaving allergens in particles form. The solvent may be, for instance, water or ethanol, according to the desired evaporation time. Other solvents may be chosen by the skilled person. This type of process to apply substances on patch backing allows nano-sized and mono-sized particles with a regular and uniform repartition of particles on the backing. This technique is adapted to any type of patch such as patch with backing comprising insulating polymer, doped polymer or polymer recovered with conductive layer. Preferably, the backing comprises a conductive material.

In another embodiment, the periphery of the backing is covered with a dry hydrophilic polymer, capable of forming an adhesive hydrogel film by contact with the moistured skin (as described in WO2009/050403). In this embodiment, the skin has to be moistured before the application of the patch. When the hydrogel comes into contact with the moistured skin, the polymer particles absorb the liquid and become adhesive, thereby creating a hermetically closed chamber when the patch is applied on the skin. Examples of such hydrogels include polyvinylpyrolidone, polyacrylate of Na, copolymer ether methyl vinyl and maleic anhydride.

The backing may be rigid or flexible, may or may not be hydrophilic, and may or may not be translucent, depending on the constituent material. In the case of glass, the support may be made break-resistant by bonding a sheet of plastic to the glass.

In one embodiment, the backing of the patch contains a transparent zone allowing directly observing and controlling the inflammatory reaction, without necessarily having to remove the patch. Suitable transparent materials include polyethylene film, polyester (polyethylene-terephtalate) film, polycarbonate and every transparent or translucent biocompatible film or material.

In a particular embodiment, the portion of the backing bearing the allergen is not in direct contact with the skin. In this embodiment, the height of the chamber defined by the backing, the periphery of the backing and the skin is in the range of 0,1 mm to 1 mm.

The method of the invention typically involves the repeated application of a device according to the invention to the subject as disclosed above, leading to a progressive decrease of the skin reactivity in the subject.

The specific dose of allergen as well as the number of applications and duration of contact can be adapted by the skilled artisan, depending on the subject, the nature of the allergen preparation, the type of patch device used, etc.

Generally, the method comprises the application of at least two patch devices as disclosed above, preferably at least 3, 5, 10 or 15, over a period of time comprised between a week and months/years. The treatment may be stopped at any time, e.g., once an eczema has been treated.

The amount of composition allergens on each patch is typically in the range of 0.1 to 1000 µg/cm of patch surface, preferably in the range of 20 to 500 µg/cm² of patch surface, more preferably in the range of 20 to 200 µg/cm² of patch surface. The patch surface is in the range of 1 cm² to 10 cm², preferably in the range of 1 cm² to 5 cm².

For application, the patch devices may be applied directly to the skin, without any pre-treatment, preferably on a hairless part of the body. Alternatively, the skin may be treated prior to application of the device, to disrupt the stratum corneum, to remove hairs or simply to cause hydration of the skin, at the site of contact with the patch device.

As disclosed in the experimental section, the method of the invention results in a progressive decrease of the skin reactivity of the subject.

Patient having an eczema due to several allergens may be treated by the epicutaneous application of several skin patch devices, each containing a specific allergen composition, and/or by the application of a device comprising a combination of these allergens. In this regard, in a particular embodiment, for treating eczema in a patient allergic to peanut and house dust mite (HDM), 2 devices may be applied by the epicutaneous route, one containing a peanut allergen composition and the other containing an HDM allergen composition. When several patches comprising distinct allergens are used, they may be applied simultaneously or sequentially, or both. Typically, they are applied under conditions allowing a contact with the skin during a common period of time.

The present invention also relates to the use of a skin patch device as described above, in the manufacture of a composition for treating eczema in a subject.

The following examples are given for purposes of illustration and not by way of limitation.

### EXAMPLES

### Example 1

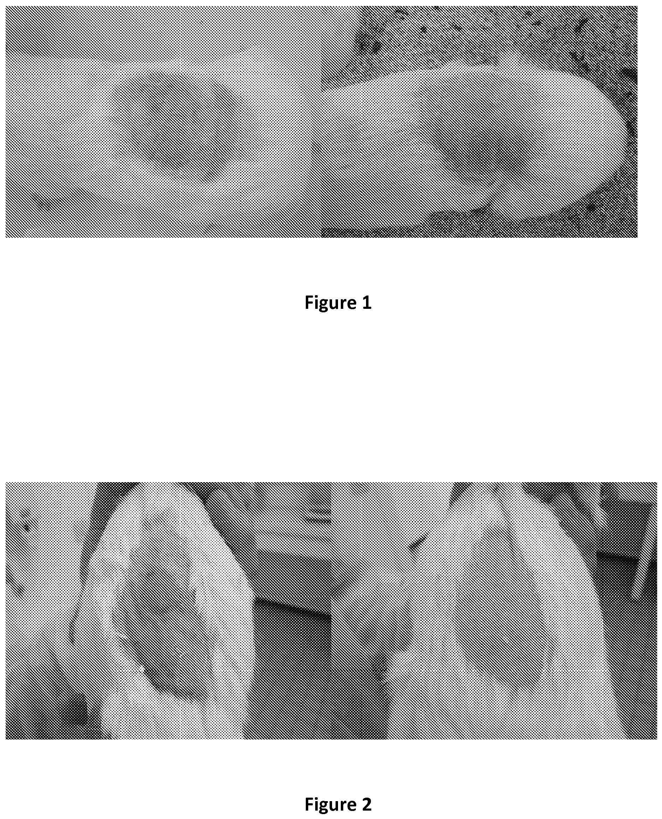

In an experiment on Guinea pigs sensitized to peanut proteins, the 48h application on the back of the animal of 100µg of peanut protein extracts (PPE) is responsible of skin lesions from the first application of PPE. These lesions (figure 1) are typically local eczema lesions with edema, erythema and vesicles. Histologically, there is a wide inflammation with macrophages and lymphocytes. Eosinophils are also noticed. During the course of the treatment, level of local reactivity decreases progressively and was completely vanished, after 3 weeks of treatment. Skin reactivity to PPE decreased dramatically in the subcutaneous tissues.

Moreover, after 3 months of treatment, atopy patch test with cow milk proteins applied on another skin area (stomach) did not provoke any reactions while, in sensitized untreated animals, a local eczematous reaction has been noticed (figure 2).

### Example 2

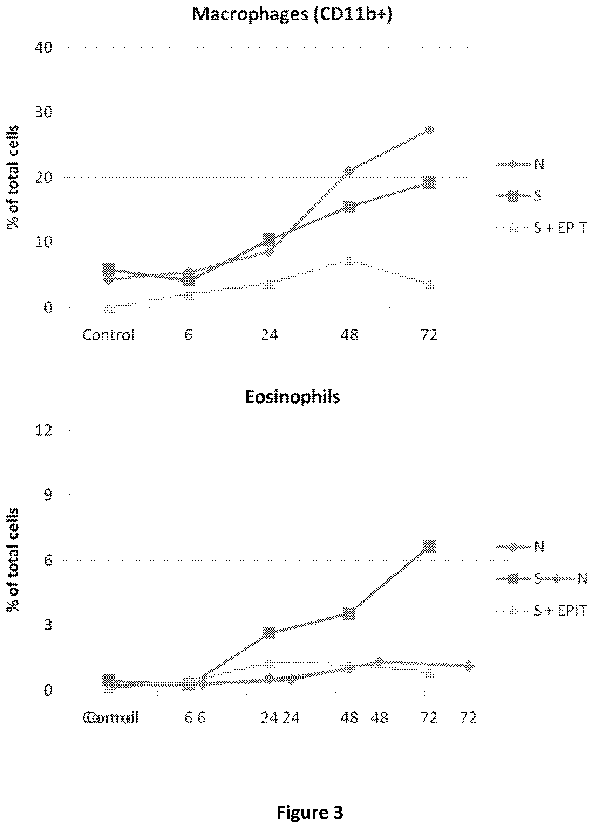

This local eczematous reaction phenomenon has also been found in a model Balb/c mice sensitized to ovalbumin (OVA) (figure 3). In this trial, two groups of sensitized mice (one treated by weekly application of Viaskin with OVA, one sensitized and not treated) were compared to a control group of non treated and non sensitized mice. After 8 weeks, an atopy patch test with OVA conjugated to fluorochrome was applied on the back during 48h. When epiderm and derm are studied, the concentration of macrophages of eosinophils fixing labeled OVA was dramatically decreased in treated animals comparing to sensitized but non treated. Thus, in this experiment histological changes are in accordance to clinical findings. Thus, OVA-sensitized mice treated by EPIT loose all reactivity to OVA during patch testing.

### Example 3

In a pilot study undertaken in cow's milk allergic children, the decrease of skin reactivity to milk during patch testing have been observed. In this trial, 16 children were included. All of them suffered from a severe cow's milk allergy with high level of specific IgE in the serum. Treatment consisted of the application for 48 hours of 1 EPIT device (Viaskin® DBV Technologies SA, Pépinière Paris Santé Cochin, Paris, France), 3 times a week, on the back, for the whole duration of the study (i.e. 3 months).

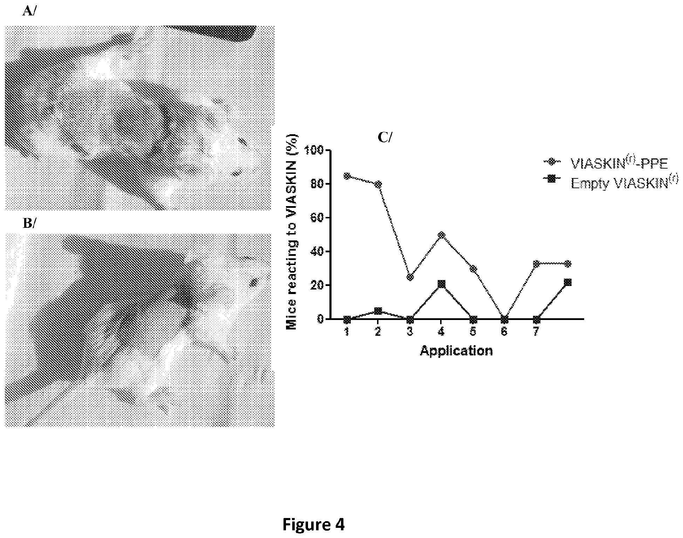

The device, equipped with the 1 mg of cow's milk, was placed in the back of the child, without any specific preparation of the skin, in 6 places previously defined in the inter-scapular area, following a clockwise order. They were maintained for 48 hours before being removed by the parents. Once applied on the skin, the device containing the milk triggers a local eczematous reaction, in the form of redness visible from the 3rd hour following application through the transparent plastic membrane, followed by the onset of a local inflammatory reaction. In the course of the treatment, the skin reactivity decreased progressively in some children as it is highlighted in the figure 4.

### Conclusion

Epicutaneous route displays potent and original way of treating eczema. Epicutaneous immunotherapy led to a decrease of the skin reactivity of treated children.

These data confirm for the first time that:
- specific epicutaneous immunotherapy by Viaskin method is able to minimize skin lesions of eczema provoked locally by skin application of allergen as well as in animal model and in patients;
- when specific epicutaneous immunotherapy has been followed, skin reactivity after allergen exposure is diminished even if the part of the skin exposed to the allergen has not been previously treated;
- when specific epicutaneous immunotherapy has been followed, inflammatory infiltration in the sub cutaneous tissues characterized by eosinophils and macrophages decrease dramatically after patch testing;
- during specific epicutaneous immunotherapy the decrease of the skin reactivity is able to determine the state of sensitivity of the patient to the allergen.

### REFERENCES

Klüken H, Wienker T, Bieber T (2003). Allergy 58 (1): 5-12.
Van Odijk J, Kull I, Borres MP, et al (2003). Allergy 58 (9): 833-43.
Jasek, W, ed (2007) (in German). Austria-Codex (62 ed.). Vienna, pp. 2720, 6770.
Beattie PE, Finlan LE, Kernohan NM, Thomson G, Hupp TR, Ibbotson SH (2005). Br. J. Dermatol. 152 (5): 1001-8.
Meduri NB, Vandergriff T, Rasmussen H, Jacobe H (2007). Photodermatol Photoimmunol Photomed 23 (4): 106-12.
Jans J, Garinis GA, Schul W, et al (2006). Mol. Cell. Biol. 26 (22): 8515-26.
Akdis et al. J Allergy Clin Immunol 2007; 119(4):780-91.
Hamelmann et al. Am J Respir Crit Care Med 1997; 156(3 Pt 1):766-75.
King et al., Bulletin of the World Health Organization, 1994, 72(5): 796-806.
Lagranderie et al. J Allergy Clin Immunol 2008; 121(2):471-8.
Pajno et al., J Allergy Clin Immunol. 2005 Dec;116(6):1380-1.
Weber, Curr Allergy Asthma Rep. 2004 Sep;4(5):401-8.
Werfel, Allergy 2006, 61:202-205.

## Claims

1. A selected allergen comprised in a skin patch device, for use in a method of treating eczema in a subject by repeated application of the device to a non eczematous intact area of the skin of the subject, wherein said device is applied under conditions allowing contact between the allergen and the skin and allowing the allergen to penetrate into stratum corneum of the epidermis, said selected allergen causing a cutaneous inflammatory reaction, said allergen being applied in dry form and in the absence of adjuvant, said application causing a progressive decrease of the skin reactivity thereby leading to a disappearance of eczema, and wherein said selected allergen is a milk, peanut, egg, pollen or dust mite allergen.

2. The selected allergen for use of claim 1, wherein a combination of allergens is applied to the skin, simultaneously or sequentially.

3. The selected allergen for use according to claim 1 or 2, wherein said eczema is atopic eczema.

4. The selected allergen for use according to any one of claims 1 to 3, wherein the device is an occlusive skin patch device comprising a composition comprising said selected allergen, and wherein said selected allergen in the patch is in dry form, devoid of an adjuvant, and adheres to the patch through electrostatic forces.

5. The selected allergen for use according to claim 4, wherein the composition further contains a pharmaceutically acceptable carrier.

## Patentansprüche

1. Ein ausgewähltes Allergen umfasst in einer Hautpflastervorrichtung zur Verwendung in einem Verfahren zum Behandeln von Ekzem in einem Subjekt durch wiederholte Anwendung der Vorrichtung auf eine nicht ekzematöse intakte Fläche der Haut des Subjekts, wobei die Vorrichtung unter Bedingungen angewendet wird, die den Kontakt zwischen dem Allergen und der Haut erlaubt und das Penetrieren in das Stratum corneum der Epidermis erlaubt, wobei das ausgewählte Allergen eine kutane entzündliche Reaktion verursacht, das Allergen in trockener Form angewendet wird und in der Abwesenheit eines Adjuvanz, diese Anwendung einen fortschreitenden Rückgang der Hautreaktivität verursacht, dadurch zu einem Verschwinden des Ekzems führt, und wobei das ausgewählte Allergen Milch-, Erdnuss-, Ei-, Pollen- oder Hausstaubmilbe-Allergen ist.

2. Das ausgewählte Allergen zur Verwendung nach Anspruch 1, wobei eine Kombination von Allergenen auf die Haut, simultan oder sequentiell, angewendet wird.

3. Das ausgewählte Allergen zur Verwendung nach Anspruch 1 oder 2, wobei das Ekzem ein atopisches Ekzem ist.

4. Das ausgewählte Allergen zur Verwendung nach einem der Ansprüche 1 bis 3, wobei die Vorrichtung eine okklusive Hautpflastervorrichtung ist umfassend eine Zusammensetzung umfassend das ausgewählte Allergen, und wobei das ausgewählte Allergen in dem Pflaster in trockener Form vorliegt, frei von einem Adjuvanz, und an das Pflaster durch elektrostatische Kräfte adhäriert.

5. Das ausgewählte Allergen zur Verwendung nach Anspruch 4, wobei die Zusammensetzung weiter einen pharmazeutisch verträglichen Träger enthält.

## Revendications

1. Allergène sélectionné compris dans un dispositif patch cutané, pour utilisation dans une méthode de traitement de l'eczéma chez un sujet par application répétée du dispositif sur une zone intacte non eczémateuse de la peau du sujet, ledit dispositif étant appliqué dans des conditions permettant le contact entre l'allergène et la peau et permettant à l'allergène de pénétrer dans la couche cornée de l'épiderme, ledit allergène sélectionné causant une réaction inflammatoire cutanée, ledit allergène étant appliqué sous forme sèche et en l'absence d'adjuvant, ladite application causant une diminution progressive de la réactivité de la peau conduisant ainsi à une disparition de l'eczéma, et ledit allergène sélectionné étant un allergène de lait, d'arachide, d'oeuf, de pollen ou d'acarien.

2. Allergène sélectionné pour utilisation selon la revendication 1, où une combinaison d'allergènes est appliquée sur la peau, simultanément ou séquentiellement.

3. Allergène sélectionné pour utilisation selon la revendication 1 ou 2, où ledit eczéma est un eczéma atopique.

4. Allergène sélectionné pour utilisation selon l'une quelconque des revendications 1 à 3, où le dispositif est un dispositif patch cutané occlusif comprenant une composition comprenant ledit allergène sélectionné, et où ledit allergène sélectionné dans le patch est sous forme sèche, sans adjuvant, et adhère au patch par des forces électrostatiques.

5. Allergène sélectionné pour utilisation selon la revendication 4, où la composition comprend en outre un support pharmaceutiquement acceptable.
